# EUROPEAN PATENT APPLICATION

(11) **EP 0 664 130 A1**
(43) Date of publication of application: **26.07.1995**
(21) Application number: 92921785.9
(22) Date of filing: 21.10.1992
(51) Int. Cl.: A61K 31/71, A61K 31/365

(54) **EXTERNAL PREPARATION FOR TREATING HEMORRHOIDAL DISEASES**

(30) Priority: 25.10.1991 JP 343869/91
(71) Applicant: Senju Pharmaceutical Co., Ltd., Osaka-shi, Osaka 541 (JP)
(72) Inventor: OGATA, Kazumi B-701, Toyonaka-shi Osaka 565 (JP)
(74) Representative: von Kreisler, Alek, Dipl.-Chem.
(86) International application number: JP9201373
(87) International publication number: WO9307884

(57) **Abstract**

A novel, useful external preparation for treating hemorrhoidal diseases which contains a polyene macrolide fungicide such as pimaricin, nystatin or amphotericin B. It is useful as a remedy for hemorrhoidal diseases, because it has an excellent effect of curing various hemorrhoidal diseases such as anal fissure, periproctitis, hemorrhoid, or anal fistula, in particular, the last two which are thought to be grave among others.

## Description

### Technical Field

This invention relates to a novel, useful external preparation for treating hemorrhoidal diseases which contains a polyene macrolide fungicide.

### Background Art

Hemorrhoidal diseases include anal fissure, periproctitis, hemorrhoid, anal fistula and so on. It is said that once people contract these diseases, the hemorrhoidal diseases hardly improve, if worsen, and therefore they are grave among others. It is said that anal fistula, which is thought to be the most grave of these hemorrhoidal diseases, repeats the suppuration and the surrounding part of the anus turns sore because of the suppurative juice which oozes out and that in most cases it is accompanied with proctocele.

Thus far, preparations for treating hemorrhoidal diseases have been on the market in various forms ranging from oral drugs to external preparations such as suppositories and ointments, and they are selected properly according to the clinical conditions. Oral preparations are used chiefly for improving action of the bowels, treating blood congestion, bleeding, swelling and pain in the affected part, and for preventing infections. In case oral preparations do not show enough action against hemorrhoidal diseases, external preparations such as suppositories or ointments are employed in combination. Suppositories and ointments are locally applied for the purposes of alleviating pain, itching, swelling, inflammation and bleeding in the affected part. Their main ingredients include, among others, antiinflammatory agents, analgesics, hemostatics and fungicides.

However, from a viewpoint of their pharmacological action, theoretically these preparations for treating hemorrhoidal diseases are supposed to show effectiveness against them. But it is the current situations that these preparations in practice do not exhibit satisfactory activities. Accordingly, the development of preparations for treating hemorrhoidal diseases having sufficient and practical curing effects has been asked for.

Under the circumstances, the present inventor conducted an extensive investigation in search of an excellent agent for treating hemorrhoidal diseases and found that a polyene macrolide fungicide showed an unexpected, excellent effect against hemorrhoidal diseases when it was administered to hemorrhoidal patients. The present invention is based on the above finding.

### Disclosure of Invention

The present invention is, therefore, directed to a novel, useful external preparation for treating hemorrhoidal diseases which contains a polyene macrolide fungicide.

The polyene macrolide fungicide employed for the external preparation for treating hemorrhoidal diseases of the invention includes, among others, pimaricin, amphotericin B, trichomycin, nystatin and pentamycin, but it is not limited to these drugs. These agents may be used singly or, if necessary, two or more species of the agents can be adequately used in combination.

The external preparation for treating hemorrhoidal diseases of the invention can be produced by conventional methods. In the case of ointment, to ointment bases such as hydrophilic ointment are added preservatives such as esters of para-oxiaminobenzoic acid and emulsifiers (surfactants) such as sodium lauryl sulfate if necessary. Then, to the resulting preparation is added the above fungicide and it is uniformly admixed. Hereinafter the intended ointment can be prepared by known methods. In the case of suppository, to bases such as cacao butter and laurin butter are added emulsifiers (surfactants), suspending agent and preservative and so on, if necessary. Then, to the resulting preparation is added the above fungicide and it is uniformly admixed. Hereinafter the intended suppository can be prepared by conventional methods (ex. melting method).

As is clear from the Experimental Examples mentioned below, the external preparation for treating hemorrhoidal diseases of the invention shows an excellent effect of curing hemorrhoidal diseases such as anal fissure, periproctitis, hemorrhoid and anal fistula, in particular, the last two which are thought to be grave among others and therefore can be employed for the treatment of hemorrhoidal diseases.

The external preparation for treating hemorrhoidal diseases of the invention can be administered preferably in the form of ointments or suppositories. The form of creams and aqueous sprays is also convenient for the patients. The suppository is inserted into the affected part (anus), and the ointment is applied to the affected part. In the case of suppositories, 0.5 g to 1 g of a suppository which contains about 1 to 500 mg of the above fungicide, preferably about 10 to 200 mg may be administered once to several times daily. In the case of ointments, 0.5 g to 1 g of an ointment in which the concentration of the fungicide is in the range of 0.1 to 5 w/w% may be administered once to several times daily. The exact dosage is, however, determined properly according to the fungicide to be employed, the patient's age or weight, the clinical conditions, the administration mode and the frequency of administration.

Unless contrary to the object of the invention, the external preparation for treating hemorrhoidal diseases of the invention may further contain other agents for treating hemorrhoidal diseases, among others, bactericide, antiinflammatory agents such as lysozyme chloride and glycyrrhizic acid, analgesics, hemostatics, agents for improving peripheral bloodstream disorder such as tocopherol acetate and agents for promoting healing of wounds such as lithospermi radix essence. Painkillers such as procaine hydrochloride, dibucaine hydrochloride and p-aminobenzoic acid ethyl ester can be adequately incorporated. Drugs having other kind of efficacy can also be adequately added.

The following examples are intended to further illustrate the invention and the experimental examples demonstrate the effect of the present invention, but the scope of the invention should not be limited by these examples.

### Best Mode for Carrying Out the Invention

### Example 1 Pimaricin ointment

Two grams (potency) of pimaricin and 98 g of hydrophilic ointment were uniformly admixed and 5 g of the preparation were filled respectively into aluminum-laminated tubes to give 20 pimaricin ointments.

### Example 2 Amphotericin B suppository

Ten amphotericin B suppositories were produced by admixing 0.1 g (potency) of amphotericin B and 20 g of cacao butter in a conventional manner.

### Example 3 Pimaricin suppository

Ten pimaricin suppositories were produced by admixing 0.5 g (potency) of pimaricin and 20 g of cacao butter in a conventional manner.

### Example 4 Nystatin suppository

A total amount of 100 g of nystatin ointment was prepared by admixing 2,100,000 international units of nystatin and a hydrophilic base in a conventional manner.

### Experimental Example 1

In the normal process of the development of drugs, assessment of a potential drug is initially conducted in an animal model in which the condition approximates that of human disease, and after that a toxicological test on animals is performed and then a human clinical test follows. For now, however, there is no such animal model for human hemorrhoidal diseases. Accordingly, because the polyene macrolide, which the external preparation for treating hemorrhoidal diseases of the invention contains as the main ingredient, is a highly safe drug as a fungicide, the following clinical tests on humans were conducted for evaluation.

To each patient diagnosed with hemorrhoid or anal fistula was administered respectively about 0.5 g to 1 g of the 2 % pimaricin hydrophilic ointment prepared in Example 1 for a month by inserting the ointment into the anus with the fingertip once daily.

As a result, the amount of the bleeding which had been seen at the time of evacuation in the patient diagnosed with hemorrhoid began to decrease the next day after administration, and the bleeding stopped completely after a week. In the meantime, the suppurative juice of the patient whose disease was diagnosed as anal fistula almost stopped after 10 days, and after a month the patient almost recovered from the proctocele as well.

### Experimental Example 2

Five days after the nystatin ointment Prepared in Example 4 was administered to a 50-year-old male patient having swollen hemorrhoid accompanied with pain, the pain was alleviated and the swelling also disappeared.

The reason the external preparation for treating hemorrhoidal diseases of the invention showed an excellent effect against hemorrhoidal diseases is presumably either because hemorrhoidal diseases are involved in fungi infections or because the polyene macrolide fungicide has any other activities on the promotion of the treatment of hemorrhoidal diseases than antifungi activity.

### Industrial Applicability

The external preparation for treating hemorrhoidal diseases of the invention shows an excellent effect against hemorrhoidal diseases such as anal fissure, periproctitis, hemorrhoid and anal fistula, in particular, the last two which are thought to be grave among others, and therefore is useful for the treatment of hemorrhoidal diseases.

## Claims

1. An external preparation for treating hemorrhoidal diseases which contains a polyene macrolide fungicide.

2. The external preparation for treating hemorrhoidal diseases as claimed in claim 1 wherein the polyene macrolide fungicide is pimaricin.

3. The external preparation for treating hemorrhoidal diseases as claimed in claim 1 wherein the polyene macrolide fungicide is nystatin.
